# EUROPÄISCHE PATENTSCHRIFT

(11) **EP 2 015 697 B1**
(45) Veröffentlichungstag und Bekanntmachung des Hinweises auf die Patenterteilung: **23.03.2011**
(21) Anmeldenummer: 07724926.6
(22) Anmeldetag: 07.05.2007
(51) Int. Cl.: A61B 19/02, B65D 43/22, B65F 1/16

(54) **SAMMEL- UND ENTSORGUNGSBEHÄLTER, INSBESONDERE FÜR KANÜLEN**
COLLECTION AND DISPOSAL CONTAINER, ESPECIALLY FOR CANNULAE
CONTENANT DE COLLECTE ET D'ÉLIMINATION, EN PARTICULIER DE SERINGUES

(30) Priorität: 10.05.2006 DE 102006022018
(43) Veröffentlichungstag der Anmeldung: 21.01.2009
(73) Patentinhaber: Rigling, Heinz, D-75382 Althengstett (DE)
(72) Erfinder: RIGLING, Heinz, 75382 Althengstett (DE); HEILAND, Christoph, 82442 Wurmansau (DE)
(74) Vertreter: Behrmann, Niels
(86) Internationale Anmeldenummer: PCT/EP2007/004002
(87) Internationale Veröffentlichungsnummer: WO 2007/128543

(56) Entgegenhaltungen:
- EP-A- 0 697 344
- US-A- 5 108 029

## Beschreibung

Die Erfindung geht aus von einem Sammel- und Entsorgungsbehälter, insbesondere für Kanülen, nachfolgend kurz "Behälter" genannt.

### Stand der Technik

Ein solcher Behälter ist aus der DE 102 31 564 B3 bekannt. Er besteht aus Kunststoff und ist vorrangig zur Benutzung in der Notfallmedizin bestimmt und z. B. in Notfall-, Unfallarzt- bzw. Rettungsdienst-Koffern enthalten. Da mit ihm während eines Notfalleinsatzes nur einige wenige, jeweils vom Spritzenkörper abgezogene Kanülen, ggf. auch Venenverweilkanülen und Tupfer, für die anschließende Entsorgung durch Verbrennen zu sammeln sind, weist er eine entsprechend kleine, wenig Platz beanspruchende Baugröße und zudem eine noch zusätzlich Platz sparende elliptische Querschnittsform auf.

Zur Vorbereitung seiner Ingebrauchnahme wird auf das Behälter-unterteil ein Behälterdeckel mit einer Einwurföffnung mit einer in sie vorstehend ausgebildeten Kanülenabzugsausgestaltung und einem um eine senkrechte Achse schwenkbar auf ihm angeordneten, zweiteilig ausgebildeten Einwurföffnungsdeckel unlösbar aufgerastet. Aus letzterem kann in seiner die Einwurföffnung abdeckenden Stellung ein kappenförmiges Verschlusselement nach unten in die Einwurföffnung gedrückt werden, um sie für die Behälterentsorgung endgültig zu verschließen.

Zum Schutz der Personen, die mit diesen kontaminierten medizinischen Abfall enthaltenden Behältern umgehen, soll der endgültige Verschluss der Einwurföffnung irreversibel sein, d. h. er darf nicht wieder rückgängig gemacht werden können, zumindest nicht ohne Werkzeug oder Gewaltanwendung.

Weil es sich bei diesen Behältern um Massenartikel handelt, die deshalb preiswert sein sollten, sollten sie auch kostengünstig herstellbar sein. Etwa dadurch, dass bei ihrer Herstellung keine Teile montiert werden müssen, also z. B. weder beim Einwurföffnungsdeckel bzw. Behälterdeckel noch im Hinblick auf die Ausgestaltung dieser beiden Deckel für den endgültigen Verschluss der Einwurföffnung.

Ein Beispiel dafür kann der aus der DE 102 14 163 A1 bekannte medizinische Abfallbehälter sein. Bei ihm ist der Einwurföffnungsdeckel mit dem Behälterdeckel durch ein an ihnen randseitig ausgebildetes Filmscharnier beweglich verbunden, wobei jedoch diese Scharnierart z. B. keine stabile "Offen"- bzw. "Geschlossen"-Stellung des Einwurföffnungsdeckels ermöglicht. Durch diese Scharnierverbindung können die beiden Deckel fertigungsgünstig als nur ein Teil hergestellt werden. Und für den endgültigen, irreversiblen Verschluss der hier fast die gesamte Fläche des kreisrunden Behälterdeckels einnehmenden Einwurföffnung wird der Einwurföffnungsdeckel auf den Behälterdeckel und gleichzeitig eine am dem Filmscharnier gegen-überliegenden Rand des Einwurföffnungsdeckels gelenkig angeformte Lasche in eine Verschlusshülse am Behälterdeckelrand gedrückt. Durch hierbei wirksam werdende Rastausgestaltungen wird die Lasche unlösbar in der Verschlusshülse gehalten.

### Problemstellung

Der vorliegenden Erfindung liegt das Problem zugrunde, einen Behälter der eingangs genannten Art so weiterzubilden, dass er keine bei seiner Herstellung zu montierende Teile aufweist. Dies soll außer für den Einwurföffnungsdeckel und Behälter-deckel auch für deren für das endgültige Verschließen der Einwurföffnung vorzusehende Ausgestaltungen gelten. Dabei soll die bestimmungsgemäße Behälterbenutzung weiterhin auf besonders einfache Weise möglich sein, insbesondere soll aber das endgültige Verschließen der Einwurföffnung auf sehr bequeme und - unabhängig von der jeweils gewählten Größe von Behälter bzw. Behälterdeckel und Einwurföffnung - ebenfalls kostengünstig ausgestaltbare Weise herbeigeführt werden können und sich die Sicherheit dieses Einwurföffnungsverschlusses und seine Irreversibilität durch eine besondere Qualität auszeichnen.

Dieses Problem wird mit den Merkmalen des Patentanspruchs 1 gelöst. Vorteilhafte Weiterbildungen sind Gegenstand der Unteransprüche.

Der erfindungsgemäß weitergebildete Behälter kann kostengünstiger produziert werden als mit dem bisherig mehrteilig ausgebildeten Einwurföffnungsdeckel bzw. Behälterdeckel, da nun auch bei ihm diese beiden Deckel gelenkig miteinander verbunden sind und sie somit ebenfalls als nur ein Teil, d. h. ohne Montagearbeiten hergestellt werden können.

Dieser Vorteil bleibt auch im Hinblick darauf erhalten, dass die erfindungsgemäße Verriegelungseinrichtung für diese beiden Deckel Folgendes umfasst: Eine muldenartige Verriegelungsvertiefung mit einer an einem Abschnitt ihres Randes in sie vorstehenden Verriegelungsraste, ausgebildet im Behälterdeckel im Bereich zwischen seinem der ersten beweglichen Deckelverbindung gegenüberliegenden Randabschnitt und dem benachbarten Randabschnitt der entsprechend davon beabstandet angeordneten Einwurföffnung und eine Art Verriegelungskörper in einer Ausnehmung im Einwurföffnungsdeckel an einer der Verriegelungsvertiefung entsprechenden stellte, randseitig durch eine bewegliche Verriegelungskörperverbindung schwenkbar gehalten und manuell nach unten in eine vorbestimmte Verriegelungsstellung in der Verriegelungsvertiefung bewegbar, in welcher Stellung er sowohl im Zusammenwirken mit einem hakenartig von ihm abstehend ausgebildeten Verriegelungselement die Verriegelungsraste teilweise umgreift als auch durch ein zungenförmig an ihm ausgebildetes Verriegelungs-Sicherungselement mit der Unterseite des Einwurföffnungsdeckels unlösbar verrastet ist.

Infolge dieser erfindungsgemäß keine losen Teile mehr erfordernden Ausbildung der für die Verriegelung des Einwurföffnungsdeckels vorgesehenen Komponenten der neuartigen Verriegelungseinrichtung sind ebenfalls keine Montagearbeiten bei der Behälterherstellung mehr notwendig. Stattdessen können auch sie kostengünstig im gleichen Arbeitsgang zur Herstellung des auch zusammen mit ihnen nun nur noch ein einziges Fertigungsteil bildenden Behälterdeckels und Einwurföffnungsdeckels mit ausgebildet werden.

Weil die in einer Ausnehmung des Einwurföffnungsdeckels vorgesehene, eine gewissermaßen tastenartige Betätigung ermöglichende gelenkige Anordnung des Verriegelungskörpers, um ihn in seine Verriegelungsstellung zu überführen, nur einen nach unten gerichteten Druck mit einem Finger erfordert, kann dieser wichtige, die Behälterentsorgung vorbereitende Vorgang auf einfache Weise zielgerichtet und damit beschleunigt ausgeführt werden. Denn hierfür kann der Einwurföffnungsdeckel vorgangserleichternd auf dem Behälterdeckel aufliegen, d. h. zugeklappt sein, statt dass er zuvor, wie im erwähnten Stand der Technik gemäß der DE 102 14 163 A1, erst noch einmal mit einer Hand festgehalten werden muss, um eine hierfür mit den Fingern der anderen Hand vom Einwurföffnungsdeckel entsprechend abzuwinkelnde Verriegelungszunge in eine Verriegelungshülse - gegen den Widerstand von Rastverzahnungen - zu schieben bzw. zu drücken.

Trotz der somit einfachen und bequemen Handhabung dieser neuartigen Verrieglungseinrichtung für den Einwurföffnungsdeckel ist der damit bewirkte endgültige Verschluss der Einwurföffnung besonders sicher. Denn zum einen erfolgt die Verriegelung nicht, wie im zitierten Stand der Technik, an exponierter Stelle, d. h. leicht zugänglich außen am Rand von Behälter-deckel und Einwurföffnungsdeckel, sondern jeweils in einem von diesem Rand beabstandeten Deckelflächenbereich. Also an einer Stelle, an der die von der verriegelungseinrichtung ausgehenden Verriegelungskräfte auch gleichmäßiger auf beide Deckel wirken und sie somit auf einem größeren Flächenbereich aneinander drücken können. Dadurch wäre ein Versuch, den Einwurföffnungsdeckel eines für die Entsorgung bereits verriegelten Behälters wieder zu öffnen, noch schwieriger.

Als eine weitere Maßnahme zur zusätzlichen Sicherung des endgültigen Verschlusses der Einwurföffnung könnte am Rand des Behälterdeckels, bzw. an einem vorzugsweise davon nach unten abstehenden Randkragen, eine den Rand des Einwurföffnungs-deckels und ggf. des Behälterdeckels, bis auf die jeweiligen Randabschnitte mit den beiden beweglichen Deckelverbindungen bzw. demjenigen mit der Greifnase, zumindest bis auf das Niveau der Oberseite des Einwurföffnungsdeckels in dessen auf dem Behälterdeckel aufliegenden Zustand, verdeckende, kragenartig vertikal hoch stehend ausgebildete Umrandung vorgesehen werden. Sie würde die übrigen Randabschnitte des Behälterdeckels mit dem darauf aufliegenden Einwurföffnungsdeckel ver-decken und somit von außen unzugänglich machen. Dadurch wäre auch ein ggf. nach dem endgültigen Einwurföffnungsverschluss zwischen diesen beiden Deckeln verbleibender oder sich auch erst später ausbildender Spalt im Auflagebereich beider Deckel, insbesondere zwischen ihren Rändern im Bereich ihrer Nebenscheitel, ebenfalls nicht zugänglich und könnte somit keinen Anreiz etwa für evtl. Versuche bieten, den geschlossenen Einwurföffnungsdeckel nachträglich wieder zu öffnen.

Unterstützt werden könnte diese zusätzliche Sicherungsmaßnahme noch durch z. B. zwei randnah ausgebildete Verstärkungsrippen oder auch nur eine, dann vorzugsweise umlaufend randnah ausgebildete Verstärkungsrippe auf der Oberseite des Einwurföffnungsdeckels, um ihn für ein rundum planes Aufliegen auf dem Behälterdeckelrand zusätzlich auszusteifen, um so die zuvor erwähnte evtl. Spaltbildung zwischen ihren von der gelenkigen Deckelverbindung und der Verriegelungseinrichtung entfernteren Randabschnitten, also insbesondere im Bereich ihrer Nebenscheitel, zu verhindern.

Die bereits durch die vom Rand beider Deckel beabstandete Anordnung der erfindungsgemäßen Verriegelungseinrichtung verbesserte Deckel-Verschlusssicherheit wird zusätzlich noch dadurch erhöht, dass der nun erfindungsgemäß herbeiführbare Verriegelungszustand des Einwurföffnungsdeckels am Behälterdeckel durch eine auch von außen unzugängliche und damit unlösbare Verrastung eines dafür ebenfalls am Verriegelungskörper zungenförmig ausgebildeten Sicherungselements an der Unterseite des Einwurföffnungsdeckels zusätzlich gesichert wird. Nämlich mittels eines die Verriegelungsraste des Behälterdeckels zum Teil von unten her, also von außen unzugänglich, umgreifenden Verriegelungselements des Verriegelungskörpers, d. h. nicht mittels einer ebenfalls, wie schon die Verriegelung selbst, außen am Rand von Behälterdeckel und Einwurföffnungsdeckel erfolgenden Sicherungs-Rastverzahnung einer Verrieglungszunge in einer dort platzierten Verriegelungshülse, wie beim aus der DE 102 14 163 A1 bekannten Abfallbehälter.

Diese wegen Unzugänglichkeit von außen nun nicht mehr überwindbare Sicherung der Verriegelung des Einwurföffnungsdeckels mit dem Behälterdeckel für die Behälterentsorgung führt zu der angestrebten besonderen Qualität der Irreversibilität des erfindungsgemäß endgültigen Verschlusses der Einwurföffnung.

Dabei signalisiert der Verriegelungskörper in seiner nach unten in die Verriegelungsausnehmung gedrückten verriegelten Stellung, in der seine Oberseite, statt schräg nach oben weisend, gut sichtbar plan zur Fläche des Einwurföffnungsdeckels verläuft, dass der Behälter für die Entsorgung verschlossen, also nicht mehr weiter benutzbar ist.

Vorzugsweise weist auch ein erfindungsgemäß ausgebildeter Behälter für eine schmale Behälterform bei kleiner Bauweise eine elliptische Querschnittsform auf. Denn dann kann die z. B. kreisrund ausgebildete Einwurföffnung ohne wesentliche Verringerung ihrer lichten Weite vorteilhaft so außermittig im entsprechend elliptischen Behälterdeckel angeordnet werden, dass Platz für die Ausbildung der erfindungsgemäß in ihm vorzusehenden muldenartigen Verriegelungsvertiefung für den Verriegelungskörper des Einwurföffnungsdeckels entsteht.

Mit Vorteil sind zusätzlich beidseits von der vorzugsweise als Filmscharnier ausgebildeten ersten beweglichen Verbindung von Behälterdeckel und Einwurföffnungsdeckel zwei stegartig ausgebildete Verbindungselemente einer zweiten beweglichen Verbindung mit schnappscharnierartigen Federeigenschaften für diese beiden Deckel vorgesehen. Sie halten den Einwurföffnungsdeckel in zwei stabilen Stellungen, nämlich einer aufgeklappten, d. h. um ca. 160° vom Behälterdeckel abgeklappten Stellung, in der die Einwurföffnung offen bzw. frei zugänglich ist, und einer fast zugeklappten Stellung, in der er mit dem Behälter-deckel einen Winkel von ca. 20° bildet.

Auch wird beim wechselweisen Einnehmen dieser beiden Stellungen der - leichte - Einwurföffnungsdeckel von der Federkraft dieser zweiten Gelenkverbindung unterstützt. Zwar muss er beim Auf- und annäherndem Zuklappen jeweils zunächst gegen die zunehmende Federkraft der sie bildenden zwei Gelenk-Spannstege bewegt werden, schließlich klappt er aber beim jeweiligen Überschreiten seiner Senkrechtstellung, ohne Zutun des Behälterbenutzers, federkraftbetätigt von selbst in die jeweilige Endstellung.

Um die Einwurföffnung vorübergehend mit dem Einwurföffnungsdeckel zu verschließen, z. B. für Behälterbenutzungspausen, wird der annähernd zugeklappte Einwurföffnungsdeckel aus seiner ca. 20°-Stellung manuell auf den Behälterdeckel gedrückt, wobei der vorzugsweise von der Unterseite des Einwurföffnungsdeckels abstehende Haftsitzkragen den insbesondere für solche Fälle vorgesehenen manuell wieder lösbaren Haftsitz im Einwurföffnungskragen einnimmt und so den Einwurföffnungsdeckel sicher auf dem Behälterdeckel festhält. Dadurch kann ein selbsttätiges, die Einwurföffnung freigebendes Aufklappen des Einwurföffnungsdeckels und ein Herausfallen von in den Behälter eingeworfenen Kanülen verhindert werden. Das ist ein wichtiger Sicherheitsaspekt für diese in meistens durch Hektik gekennzeichneten Notfallsituationen benutzten Behälter, in denen sie auch einmal herunterfallen können, wobei aber der Einwurföffnungsdeckel dennoch nicht aufgehen, d. h. nicht aufklappen sollte.

Die Federkraftunterstützung der Stellungswechsel des Einwurföffnungsdeckels durch die beiden Gelenk-Spannstege kann sogar die Einhandbedienung dieses erfindungsgemäß weitergebildeten Behälters ermöglichen, da er dadurch z. B. allein mit dem an der vorzugsweise vom Rand des Einwurföffnungsdeckels abstehend ausgebildeten Greifnase anliegenden Daumen der den Behälter z. B. haltenden linken Hand aufgeklappt werden kann, um dann mit der rechten Hand an der vorzugsweise innen am Einwurföffnungskragen vorstehend ausgebildeten Kanülenabzugseinrichtung eine benutzte Kanüle vom Spritzenkörper abzuziehen. Ebenso kann er anschließend auch wieder mit dem Daumen in seine ca. 20°-Stellung geklappt werden.

### Ausführungsbeispiel

Anhand von schematischen Zeichnungen wird nachfolgend ein bevorzugtes Ausführungsbeispiel eines erfindungsgemäß ausgestalteten Sammel- und Entsorgungsbehälters näher beschrieben.

Es zeigen:
- Fig. 1: eine perspektivische Ansicht des Behälters mit i- dealisiert abgeklappt gezeigtem Einwurföffnungs- deckel;
- Fig. 1a: eine Vergrößerung der Einzelheit A in Fig. 1;
- Fig. 1b: eine Vergrößerung der Einzelheit B in Fig. 1;
- Fig. 2: eine Draufsicht auf den Behälter nach Fig. 1;
- Fig. 3: eine Schnittansicht von Behälterdeckel und Einwurf- öffnungsdeckel nach Fig. 2 entlang der Linie III- III;
- Fig. 4: die Schnittansicht nach Fig. 3 bei zugeklapptem und verriegeltem Einwurföffnungsdeckel;
- Fig. 5: eine weitere perspektivische Ansicht des Behälters nach Fig. 1 mit zugeklapptem, noch nicht verrie- geltem Einwurföffnungsdeckel;
- Fig. 5a: eine Vergrößerung der Einzelheit C in Fig. 5;
- Fig. 6: eine Längsschnittansicht des Behälters nach Fig. 5;
- Fig. 6a: eine Vergrößerung der Einzelheit D in Fig. 6;
- Fig. 7: eine der Längsschnittansicht nach Fig. 6 entspre- chende Schnittansicht des Behälters bei jedoch verriegeltem Einwurföffnungsdeckel und
- Fig. 7a: eine Vergrößerung der Einzelheit E in Fig. 7.

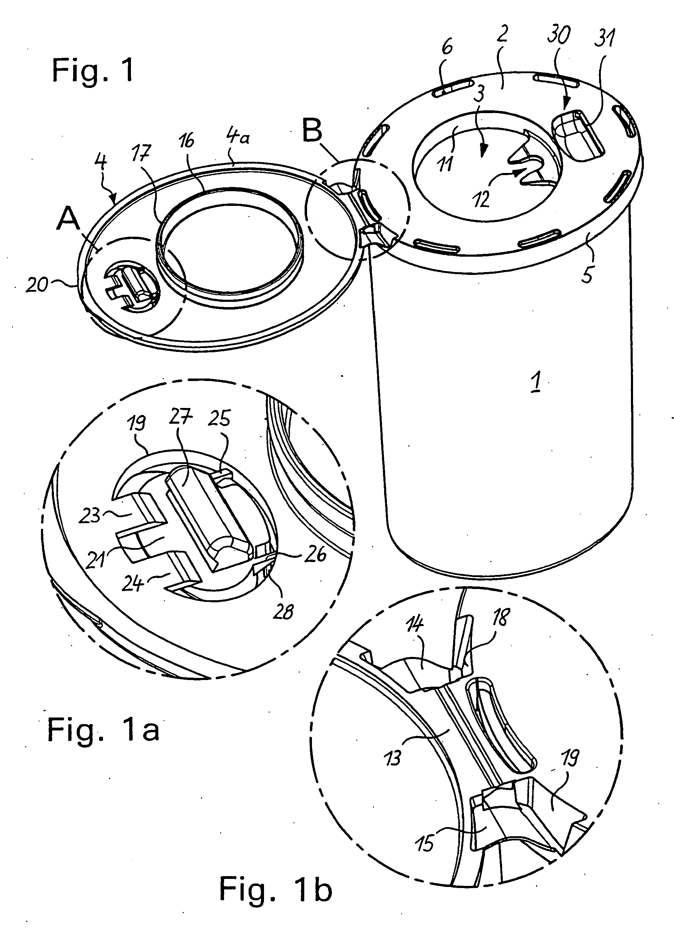

Der in den Figuren vergrößert gezeigte, vorrangig zur Benutzung in der Notfallmedizin bestimmte kleine Kanülen-Sammel- und Entsorgungsbehälter hat ein sich für Stapelzwecke leicht konisch nach unten verjüngendes Behälterunterteil 1 mit elliptischer Querschnittsform und einen zur Vorbereitung der Behälter-Ingebrauchnahme durch Aufrasten unlösbar darauf angeordneten Behälterdeckel 2 mit einer kreisrunden Einwurföffnung 3 und einem zum Auf- und annäherndem Zuklappen randseitig gelenkig mit dem Behälterdeckel 2 verbundenen Einwurföffnungsdeckel 4 (Fig. 1).

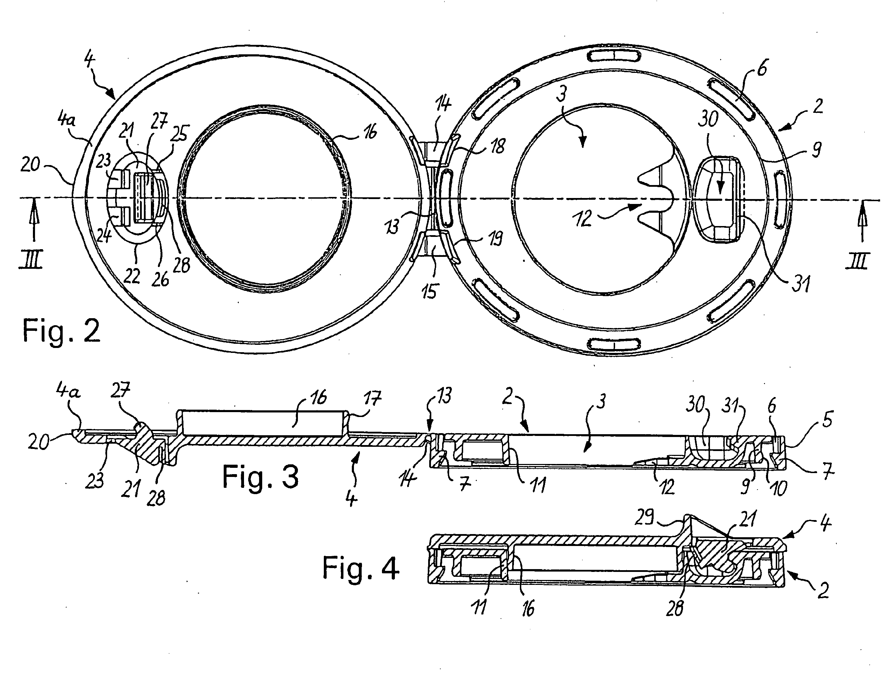

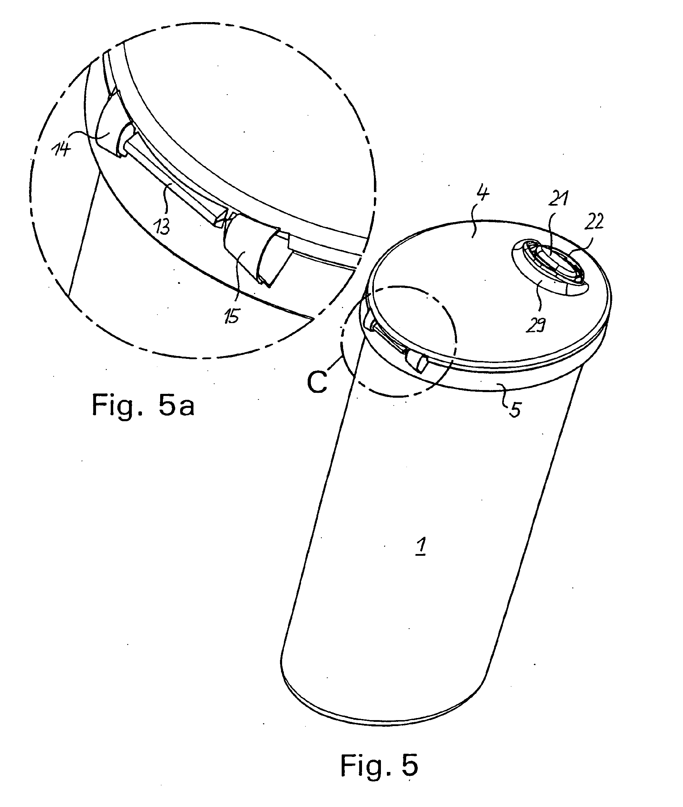

Der gezeigte Behälter hat bei vollständig zugeklapptem Einwurföffnungsdeckel 4 (Fig. 5 bis 7), in welchem dieser mit einer auf seiner Unterseite im Randbereich ausgebildeten Randverstärkungsrippe 4a (Fig. 1, 3) bestimmungsgemäß plan auf dem Behälterdeckel 2 aufliegt, eine Gesamthöhe von nur ca. 117 mm und der elliptische Behälterdeckel 2 eine Größe zwischen ca. 49 und ca. 60 mm.

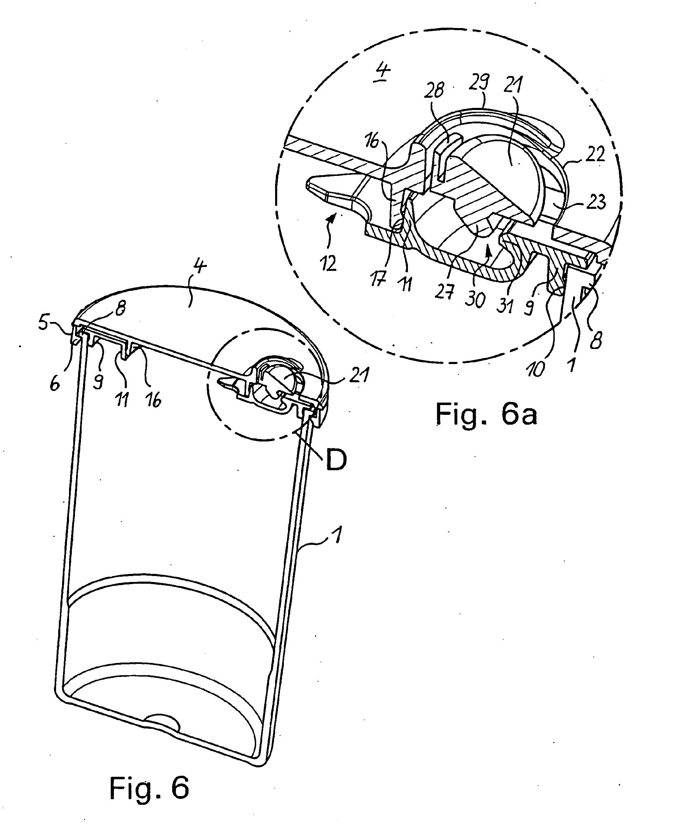

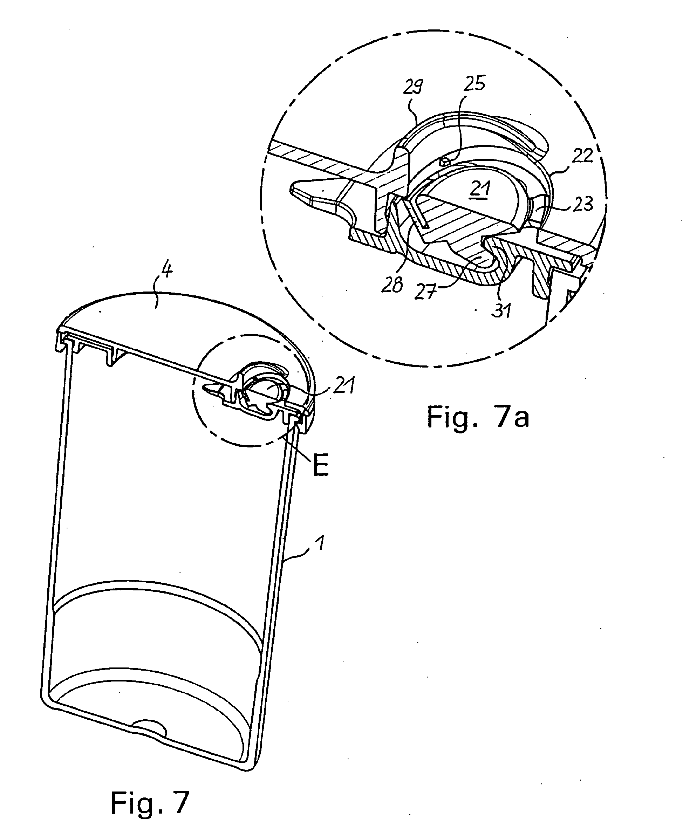

Beim Aufrasten des Behälterdeckels 2 auf das Behälterunterteil 1 sind auf der Innenseite seines nach unten abstehenden Randkragens 5 vorspringende Rastnasen 6, jeweils unterhalb der für ihre Ausbildung erforderlichen Schieberöffnungen 7, unter einer seitlich am Öffnungsrand des Behälterunterteils 1 abstehend ausgebildeten Verrastungsrippe 8 eingerastet. Ein randnah auf seiner Unterseite umlaufender Dichtungskragen 9 liegt mit einer am freien Kragenende auf der Außenseite ausgebildeten Art Dichtungswulst 10 an der Innenseite des Behälterunterteils 1 abdichtend an (Fig. 6a, 7a).

Die Einwurföffnung 3 ist leicht außermittig, d. h. nach links versetzt im Behälterdeckel 2 ausgebildet (Fig. 1, 3). Sie ist auf dessen Unterseite von einem nach unten abstehenden Einwurföffnungskragen 11 umgeben, an dessen freien Ende auf der Innenseite eine Kanülenabzugseinrichtung 12 gabelartig in die Einwurföffnung 3 vorstehend ausgebildet ist.

Der im Wesentlichen die gleiche Größe - wie auch elliptische Kontur - wie der Behälterdeckel 2 aufweisende Einwurföffnungs-deckel 4 dient sowohl dem zeitweiligen Verschluss bzw. dem zeitweiligen Abdecken der Einwurföffnung 3, z. B. in Behälterbenutzungspausen, als auch deren endgültigem Verschluss für die Behälterentsorgung. Zur gelenkigen Verbindung dieser beiden Deckel 2 und 4 sind zwei Gelenke vorgesehen: Ein, wie in Fig. 1 und 2 bzw. Fig. 1a und 2a in Vergrößerung gezeigt, erstes, als Filmscharnier 13 ausgebildetes Gelenk und ein von zwei beidseits in einem Abstand von diesem ausgebildeten Art Gelenk-Spannstegen 14 und 15 gebildetes zweites Gelenk. Dadurch bilden die beiden Deckel 2 und 4 nur ein einziges, bei der Herstellung keine Montagearbeiten erforderndes Teil.

Die Gelenk-Spannstege 14 und 15 haben die Funktion eines sich durch eine Federeigenschaft auszeichnenden Schnapp- bzw. Federscharniers. Dadurch halten sie den Einwurföffnungsdeckel 4, anders als in den Figuren idealisiert gezeigt, auf sichere Weise zum einen in einer statt um 180° nur etwa um 160° vom Behälterdeckel 2, von ihnen unterstützt, eingenommenen abgeklappten, die Einwurföffnung 3 freigebenden stabilen Stellung, zum anderen in einer in den Figuren nicht gezeigten, von ihnen ebenfalls unterstützt eingenommenen und gehaltenen stabilen "Zuklapp"-Stellung im ca. 20°-Winkel vom Behälterdeckel 2. In beiden Stellungen sind diese Gelenk-Spannstege 14 und 15 bei jeweils nur gering wirksamer, gegen eine Stellungsveränderung des Einwurföffnungsdeckels 4 gerichteter Federkraft entlastet.

An der in Fig. 1 gezeigten Unterseite des Einwurföffnungsdeckels 4 ist - in konzentrischer Anordnung zur Einwurföffnung 3 im Behälterdeckel 2 - ein nach unten abstehender Haftsitzkragen 16 mit einer auf der Außenseite seines freien Endes wulstartigen, sowohl für eine Haftwirkung als auch zur Haftsitzabdichtung bestimmten Materialverdickung 17 ausgebildet.

Zum zeitweiligen Abdecken der Einwurföffnung 3, wie schließlich auch deren endgültigen Verschluss, wird der Einwurföffnungsdeckel 4 ggf. zunächst aus seiner eingenommenen ca. 160°-Offen-Stellung gegen eine zunehmende Federkraft der Gelenk-Spannstege 14 und 15, schließlich aber, von deren Federkraft bei ihrem nach rechts Kippen unterstützt, nach rechts in die ca. 20°-"Zuklapp"-Stellung geklappt. In dieser Stellung greift sein Haftsitzkragen 16 noch nicht in den Einwurföffnungskragen 11 ein.

Überwiegend aus dieser ca. 20°-Stellung wird durch z. B. Druck mit den Fingern auf den Einwurföffnungsdeckel 4 sein Haftsitzkragen 16 in den die Einwurföffnung 3 einfassenden Einwurföffnungskragen 11 gedrückt, so dass er mit seiner Randverstärkungsrippe 4a bestimmungsgemäß plan auf dem Behälterdeckel 2 aufliegt und die Materialverdickung 17 des sich leicht konisch zu seinem freien Ende hin erweiternden Haftsitzkragens 16 an der Innenseite des sich leicht konisch nach unten verengenden Einwurföffnungskragens 11 anliegt (Fig. 4). Dadurch wird einerseits die so abgedeckte bzw. vorübergehend verschlossene Einwurföffnung 3 flüssigkeitsdicht abgedichtet, andererseits wird währenddessen auch noch der Einwurföffnungsdeckel 4 durch den dadurch herbeigeführten Haftsitz, d. h. ohne weitere Maßnahmen, gegen ein evtl. selbsttätiges Abklappen vom Behälterdeckel 2 auf diesem festgehalten.

Wie in Fig. 5 bzw. 5a gezeigt, haben dann die beiden Gelenk-Spannstege 14 und 15 infolge der für sie im Randkragen 5 des Behälterdeckels 2 ausgebildeten Ausnehmungen 18 und 19 eine stabile Endlage eingenommen, in der sie ebenfalls wieder nahezu entlastet sind.

Zum erneuten Öffnen der vorübergehend abgedeckten Einwurföffnung 3, z. B. nach einer Behälter-Benutzungspause, kann der Einwurföffnungsdeckel 4 an einer an ihm, den beiden Gelenken 13 und 14, 15 gegenüberliegend, geringfügig vorspringend ausgebildeten Greifnase 20 hochgezogen werden, damit er wieder in seine stabile ca. 160°-Offen-Stellung klappt. Und zwar zunächst wieder gegen den von der den Gelenk-Spannstegen 14 und 15 innewohnenden Federkraft bewirkten Widerstand, schließlich aber mit deren FederkraftUnterstützung.

Erst zur Entsorgung des Behälters wird die Einwurföffnung 3 endgültig, d. h. irreversibel mit dem Einwurföffnungsdeckel 4 verschlossen, indem letzterer in seiner auf dem Behälterdeckel 2 aufliegenden, die Einwurföffnung 3 abdeckenden Haftsitz-Stellung so mit dem Behälterdeckel 2 verriegelt wird, dass er zum Schutz der mit diesen Behältern umgehenden Personen nicht mehr ohne Gewaltanwendung oder Werkzeug aufgeklappt, d. h. die Einwurföffnung nicht wieder geöffnet werden kann. Hierfür sind an Behälterdeckel 2 und Einwurföffnungsdeckel 4 entsprechend zusammenwirkende Komponenten einer Verriegelungseinrichtung ausgebildet.

Dabei handelt es sich beim Einwurföffnungsdeckel 4 um einen kleinen, z. B. mit der Spitze eines Fingers tastenartig zu betätigenden Verriegelungskörper 21 mit einer elliptischen Kontur und einer Größe etwa in der Größenordnung einer größeren Tablette. Er ist in einer geringfügig größeren, in einer den Gelenken 13 bzw. 14, 15 gegenüberliegenden Anordnung, randseitig der Einwurföffnung 3 benachbart platzierten, ebenfalls elliptisch ausgebildeten Ausnehmung 22 für die Dauer des Kanülensammelns unbeweglich gehalten. Und zwar von zwei Gelenk-stegen 23 und 24 und von zwei, diesen gegenüberliegenden, mehr oder weniger punktförmig ausgebildeten Materialbrücken 25 und 26, die ihn bis zum Verriegelungszeitpunkt in der Ausnehmung 22 arretieren.

Wie in Fig. 6a zu erkennen ist, hat der Verriegelungskörper 21 im gezeigten unverriegeltem Zustand in seiner von der Hauptachse des elliptischen Einwurföffnungsdeckels 4 definierten Mitte - in vertikaler Schnittrichtung gesehen - eine, von der Ausgestaltung an seiner Unterseite abgesehen, im wesentlichen dreieckige Querschnittsfläche, die symmetrisch von seinen beiden Hauptscheitelrändern zur Mitte hin an Größe zunimmt, so dass er etwa einem Kugelkeil ähnlich sieht.

An seiner Unterseite ist ein quer zur Hauptachse des Einwurföffnungsdeckels 4 verlaufendes Verriegelungselement 27 in Form eines hakenartig schräg nach unten rechts weisenden Materialvorsprungs und an seinem seinen Gelenkstegen 23 und 24 mittig gegenüberliegenden Randabschnitt ein zungenförmig hoch stehendes Verriegelungs-Sicherungselement 28 ausgebildet.

Als eine Art "Auslöseschutz" für den Verriegelungskörper 21 ist die sozusagen innenliegende Randhälfte der Ausnehmung 22 von einem beidseits zur Hauptachse des Einwurföffnungsdeckels 4 hin symmetrisch ansteigend ausgebildeten Auslöse-Schutzkragen 29 umgeben (Fig. 6a).

Im Behälterdeckel 2 ist als mit dem Verriegelungskörper 21 zusammenwirkende Komponente der Verriegelungseinrichtung eine für dessen teilweise Aufnahme entsprechend muldenartige Verriegelungsvertiefung 30 - bei auf ihm aufliegendem Einwurföffnungsdeckel 4 - direkt unterhalb von ihm ausgebildet. In ihrem dem nahe gelegenen Hauptscheitel des Behälterdeckels 2 benachbarten Randbereich weist sie eine, rechtwinklig zur Hauptachse des Einwurföffnungsdeckels 4 verlaufend, in sie vorstehend ausgebildete Art Verrieglungsraste 31 auf. Ihr dieser gegen-überliegender, nahe bis an den Rand der Einwurföffnung 3 heranreichender, konvex gekrümmt ausgebildeter Randabschnitt befindet sich bei aufliegendem Einwurföffnungsdeckel 4 unterhalb von dessen Auslöse-Schutzkragen 29.

Zum Verriegeln des Einwurföffnungsdeckels 4 mit dem Behälterdeckel 2, auf dem er aufliegt, für den endgültigen Verschluss der Einwurföffnung 3 für die Behälterentsorgung, wird der Verriegelungskörper 21, wie oben schon erläutert, mit einer Fingerspitze tastenartig unten gedrückt, so dass er zunächst von den beiden schwach ausgebildeten Materialbrücken 25 und 26 abreißt. Anschließend schwenkt er, durch die beiden Gelenkstege 23 und 24 geführt, nach unten, bis der Verriegelungskörper 21 oben und das hakenartig an seiner Unterseite ausgebildete Verriegelungselement 27 unten an der Verriegelungsraste 31, sie gemeinsam klauenartig zum Teil umgreifend, anliegen.

Gleichzeitig ist dann das zunächst beim Schwenken des Verriegelungskörpers 21 nach unten durch Entlanggleiten am Rand der Ausnehmung 22 (unterhalb des Auslöse-Schutzkragens 29) zunehmend unter Spannung an den Verriegelungskörper 21 heran gedrückte zungenförmige Verriegelungs-Sicherungselement 28 in seine in Fig. 4 bzw. 7 und 7a gezeigte unlösbare Verrastungsstellung an der Unterseite des Einwurföffnungsdeckels 4 unterhalb des Auslöse-Schutzkragens 29 gesprungen.

Diese doppelte Funktion des verriegelns des Einwurföffnungsdeckels 4 mit dem Behälterdeckel 2 und der Verriegelungssicherung des Verriegelungskörpers 21 aufgrund des Zusammenwirkens von Verriegelungselement 27 einerseits und Verriegelungs-Sicherungselement 28 andererseits ermöglicht auf besonders zuverlässige Weise die Irreversibilität dieser Verrieglung und damit den endgültigen Verschluss der Einwurföffnung 3 für die Behälterentsorgung. Denn das Verriegelungs-Sicherungselement 2.8 ist in seiner Verrastungsstellung sowohl überwiegend vom Einwurföffnungsdeckel 4 verdeckt als auch vom restlichen Verriegelungskörper 21 mit seiner etwas unterhalb der Oberseite des Einwurföffnungsdeckels 4 plan zu dieser verlaufenden Oberseite geschützt. Damit ist es von außen unzugänglich und folglich nicht mehr aus seiner Verrastung an der Unterseite des Einwurföffnungsdeckels 4 herauslösbar.

Dadurch ist dieser einfache, auf bequeme, aber zügigzielge-richtete Weise nur mit einer Fingerbewegung herbeiführbare-endgültige Verschluss der Einwurföffnung 3 nach hierzu erfolgter Verriegelung des Einwurföffnungsdeckels 4 durch das Verriegelungselement 27 und der Sicherung dieser Verriegelung des Einwurföffnungsdeckels 4 durch das Verriegelungs-Sicherungselement 28 des Verriegelungskörpers 21 besonders sicher gegen evtl. nachträgliche Öffnungsversuche geschützt. Auf diese Weise werden alle diesbezüglichen Anforderungen erfüllt, und zwar ohne dass die hierfür an den beiden Deckeln 2 und 4 zusammenwirkend vorgesehenen Komponenten 21 bis 31 der erfindungsgemäßen Verriegelungseinrichtung Montagearbeiten bei der Herstellung des Behälters erfordern würden. Stattdessen ist auch erfindungsgemäß diese Verriegelungseinrichtung, bzw. die sie bildenden Komponenten 21 bis 31, zusammen mit dem Behälterdeckel 2 und dem mit ihm gelenkig verbundenen Einwurföffnungsdeckel 4, Teil eines einzigen, in einem Arbeitsgang ohne Montagearbeiten entsprechend kostengünstiger herstellbaren Fertigungsteils.

### Bezugszeichenliste

- 1: Behälterunterteil
- 2: Behälterdeckel
- 3: Einwurföffnung
- 4: Einwurföffnungsdeckel
- 4a: Randverstärkungsrippe
- 5: Randkragen
- 6: Rastnasen
- 7: Schieberöffnung
- 8: Verrastungsrippe
- 9: Dichtungskragen
- 10: Dichtungswulst
- 11: Einwurföffnungskragen
- 12: Kanülenabzugseinrichtung
- 13: erste bewegliche Deckelverbindung (Filmscharnier)
- 14, 15: zweite bewegliche Deckelverbindung (Gelenk- Spannstege)
- 16: Haftsitzkragen
- 17: Materialverdickung
- 18: Ausnehmung
- 19: Ausnehmung
- 20: Greifnase
- 21: Verriegelungskörper
- 22: Ausnehmung
- 23, 24: bewegliche Verriegelungskörperverbindung (Gelenk- stege)
- 25: Materialbrücke
- 26: Materialbrücke
- 27: Verriegelungselement
- 28: Verriegelungs-Sicherungselement
- 29: Auslöse-Schutzkragen
- 30: Verriegelungsvertiefung
- 31: Verriegelungsraste

## Patentansprüche

1. Sammel- und Entsorgungsbehälter, insbesondere für Kanülen, mit einem Behälterdeckel (2) mit einer Einwurföffnung (3) und einem randseitig durch zumindest eine erste bewegliche Deckelverbindung (13) auf- und zuklappbar mit ihm verbundenen, in zugeklappter Stellung durch eine Verriegelungseinrichtung mit ihm verriegelbaren Einwurföffnungsdeckel (4),
**dadurch gekennzeichnet,**
**dass** die Verriegelungseinrichtung Folgendes umfasst:
- eine muldenartige Verriegelungsvertiefung (30) mit einer an einem Abschnitt ihres Randes in sie vorstehend ausgebildeten Verriegelungsraste (31), ausgebildet im Behälterdeckel (2) im Bereich zwischen seinem der ersten beweglichen Deckelverbindung (13) gegenüberliegenden Randabschnitt und dem benachbarten Randabschnitt der entsprechend davon beabstandet angeordneten Einwurföffnung (3), und
- eine Art Verriegelungskörper (21) in einer Ausnehmung (22) im Einwurföffnungsdeckel (4) an einer der Verriegelungsvertiefung (30) entsprechenden Stelle, randseitig durch eine bewegliche Verriegelungskörperverbindung (23, 24) schwenkbar gehalten und manuell nach unten in eine vorbestimmte Verriegelungsstellung in der Verriegelungsvertiefung (30) bewegbar, in welcher Stellung er sowohl im Zusammenwirken mit einem hakenartig von ihm abstehend ausgebildeten Verriegelungselement (27) die Verriegelungsraste (31) teilweise umgreift als auch durch ein zungenförmig an ihm ausgebildetes Verriegelungs-Sicherungselement (28) mit der Unterseite des Einwurföffnungsdeckels (4) unlösbar verrastet ist.

2. Behälter nach Anspruch 1, **dadurch gekennzeichnet, dass** der Einwurföffnungsdeckel (4) im Wesentlichen die gleiche Größe und Kontur wie der Behälterdeckel (2) aufweist.

3. Behälter nach Anspruch 1 oder 2, **dadurch gekennzeichnet, dass** für die Oberseite des Behälterdeckels (2) und die daran entsprechend angepasste Unterseite des Einwurföffnungs-deckels (4) eine plane Ausgestaltung vorgesehen ist.

4. Behälter nach einem der Ansprüche 1 bis 3, **dadurch gekennzeichnet, dass** die Einwurföffnung (3) außermittig, d. h. der ersten Deckelverbindung (13) angenähert, im Behälter-deckel (2) ausgebildet ist.

5. Behälter nach einem der vorhergehenden Ansprüche, **dadurch gekennzeichnet, dass** von der Unterseite des Behälterdeckels (2) ein die Einwurföffnung (3) einfassender Einwurföffnungskragen (11) absteht, auf dessen Innenseite in einem Abstand von der Unterseite zumindest eine Kanülenabzugseinrichtung (12) in die Einwurföffnung (3) vorstehend ausgebildet ist.

6. Behälter nach einem der vorhergehenden Ansprüche, **dadurch gekennzeichnet, dass** von der Unterseite des Einwurföffnungsdeckels (4) ein Haftsitzkragen (16) in einer der Einwurföffnung (3) entsprechenden Positionierung absteht, der bei auf dem Behälterdeckel (2) aufliegendem Einwurföffnungsdeckel (9) einen manuell lösbaren Haftsitz im Einwurföffnungskragen (11) einnimmt.

7. Behälter nach einem der vorhergehenden Ansprüche, **dadurch gekennzeichnet, dass** der Verriegelungskörper (21) und die Ausnehmung (22), in der er gehalten ist, aneinander angepasste elliptische Konturen aufweisen und die Hauptachse
des Verriegelungskörpers (21) rechtwinklig zur Hauptachse
des elliptischen Einwurföffnungsdeckels (4) verläuft.

8. Behälter nach einem der vorhergehenden Ansprüche, **dadurch gekennzeichnet, dass** der Verrieglungskörper (21) annähernd die Form eines Keils ähnlich eines Kugelkeils aufweist mit in unverriegelter Stellung in vertikaler Richtung von seinen beiden Hauptscheiteln zur Mitte hin dreieckförmig in der Höhe zunehmender Querschnittsfläche.

9. Behälter nach Anspruch 8, **dadurch gekennzeichnet, dass** vom Verriegelungskörper (21) im unverriegelten Zustand die Unterseite, im verriegelten Zustand dagegen die Oberseite plan zur Oberseite des Behälterdeckels (2) verläuft.

10. Behälter nach einem der vorhergehenden Ansprüche, **dadurch gekennzeichnet, dass** das Verrieglungselement (27) als ein im unverriegelten Zustand des Verriegelungskörpers (21) von dessen Unterseite in dessen Hauptachsenrichtung verlaufender, hakenartig schräg nach unten zur Verriegelungsraste (31) weisender Materialvorsprung ausgebildet ist.

11. Behälter nach einem der vorhergehenden Ansprüche, **dadurch gekennzeichnet, dass** das Verriegelungs-Sicherungselement (28) mittig am der beweglichen Verriegelungskörperverbiridung (23, 24) gegenüberliegenden Rand des Verriegelungskörpers (21) zungenartig senkrecht hoch stehend ausgebildet ist.

12. Behälter nach einem der vorhergehenden Ansprüche, **dadurch gekennzeichnet, dass** der Verriegelungskörper (21) an seiner der beweglichen Verriegelungskörperverbindung (23, 24) gegenüberliegenden Seite von zwei beidseits des Verriegelungs-Sicherungselements (28) abreißbar ausgebildeten Materialbrücken (25, 26) für die Dauer der Behälterbenutzung mit dem Rand der Ausnehmung (22) verbunden ist.

13. Behälter nach einem der vorhergehenden Ansprüche, **dadurch gekennzeichnet, dass** am zumindest annähernd halben, dem benachbarten Rand des Einwurföffnungsdeckels (4) diametral gegenüberliegenden Rand der Ausnehmung (22) des Verriegelungskörpers (21) ein senkrecht hoch stehender Auslöse-Schutzkragen (29) ausgebildet ist mit von deren Hauptscheiteln jeweils zur Mitte des Auslöse-Schutzkragens (29) hin ansteigender Höhe.

14. Behälter nach einem der vorhergehenden Ansprüche, **dadurch gekennzeichnet, dass** die erste bewegliche Deckelverbindung (13) als ein Filmscharnier ausgebildet ist.

15. Behälter nach Anspruch 9, **dadurch gekennzeichnet, dass** der Behälterdeckel (2) und der Einwurföffnungsdeckel (4) durch eine zweite bewegliche Deckelverbindung (14, 15) miteinander verbunden sind, die von zwei beidseits des Filmscharniers (13) ausgebildeten Gelenk-Spannstegen (14, 15) mit schnappscharnierartigen Federeigenschaften gebildet ist.

16. Behälter nach Anspruch 1, **dadurch gekennzeichnet, dass** er eine elliptische Querschnittsform aufweist.

17. Behälter nach einem der vorhergehenden Ansprüche, **dadurch gekennzeichnet, dass** am Rand oder Randkragen (5) des Behälterdeckels (2) eine kragenartig vertikal hoch stehend ausgebildete, den Rand des Einwurföffnungsdeckels (4) und ggf. des Behälterdeckels (2), bis auf die jeweiligen Randabschnitte mit den beiden beweglichen Deckelverbindungen (13; 14, 15) bzw. demjenigen mit der Greifnase (20), zumindest bis auf das Niveau der Oberseite des Einwurföffnungsdeckels (4) in dessen auf dem Behälterdeckel (2) aufliegenden Zustand, verdeckende Umraridung vorgesehen ist.

## Claims

1. Collection and disposal container, in particular for cannulae, comprising a container lid (2) with a slot opening (3) and a slot opening lid (4) which is connected to said container lid at the edge so as to be closable and openable in a hinged manner by at least a first movable lid connection (13), and which can be locked on said container lid in the closed state by a locking means, **characterised in that** the locking means comprises:
- a trough-like locking indentation (30) with a locking catch (31) which is formed on a portion of the edge of said locking indentation and projects therein, formed in the container lid (2) in the region between the edge portion thereof opposite the first movable lid connection (13) and the adjacent edge portion of the slot opening (3) accordingly arranged at a distance therefrom, and
- a type of locking member (21) in a recess (22) in the slot opening lid (4) in a place corresponding to the locking indentation (30), held pivotally at the edge by a movable locking member connection (23, 24) and manually movable downwards into a predetermined locking position in the locking indentation (30), in which position it both surrounds the locking catch (31) in part in cooperation with a hook-like locking element (27) formed a protruding from said locking member, and is locked to the underside of the slot opening lid (4) in a non-detachable manner by a tongue-shaped locking safety element (28) formed on said locking member.

2. Container according to claim 1, **characterised in that** the slot opening lid (4) is of substantially the same size and shape as the container lid (2).

3. Container according to either claim 1 or claim 2, **characterised in that** a planar configuration is provided for the upper face of the container lid (2) and the underside of the slot opening lid (4) accordingly adapted thereto.

4. Container according to any one of claims 1 to 3, **characterised in that** the slot opening (3) is formed excentrically in the container lid (2), i.e. close to the first lid connection (13).

5. Container according to any one of the preceding claims, **characterised in that** a slot opening collar (11) bordering the slot opening (3) protrudes from the underside of the container lid (2), on the inner face of which slot opening collar at least one cannula withdrawal device (12) is formed projecting into the slot opening (3) at a distance from the underside.

6. Container according to any one of the preceding claims, **characterised in that** a press fit collar (16) protrudes from the underside of the slot opening lid (4), is positioned in accordance with the slot opening (3) and is press fit in the slot opening collar (11) in a manually detachable manner when the slot opening lid (9) is resting on the container lid (2).

7. Container according to any one of the preceding claims, **characterised in that** the locking member (21) and the recess (22) in which it is retained have elliptical contours adapted to one another and the main axis of the locking member (21) extends at right angles to the main axis of the elliptical slot opening lid (4).

8. Container according to any one of the preceding claims, **characterised in that** the locking member (21) is approximately wedge-shaped, similarly to a spherical wedge, with a triangular cross-sectional area increasing heightwise in the vertical direction from each of its two main peaks to the centre in the unlocked position.

9. Container according to claim 8, **characterised in that** in the unlocked state the underside extends from the locking member (21) in a planar manner relative to the upper face of the container lid (2), and by contrast in the locked state the upper face extends from the locking member (21) in a planar manner relative to the upper face of the container lid (2).

10. Container according to any one of the preceding claims, **characterised in that** the locking element (27) is formed as a material projection extending, in the unlocked state of the locking member (21), from the underside thereof in the direction of the main axis thereof and pointing downward towards the locking catch (31) in a hook-like and diagonal manner.

11. Container according to any one of the preceding claims, **characterised in that** the locking safety element (28) is formed centrally on the edge of the locking member (21) opposite the movable locking member connection (23, 24) in a tongue-like manner and standing upright.

12. Container according to any one of the preceding claims, **characterised in that**, for the duration of use of the container, the locking member (21) is connected on its side opposite the movable locking member connection (23, 24) to the edge of the recess (22) by two material bridges (25, 26) which are formed on either side of the locking safety element (28) and can be torn off.

13. Container according to any one of the preceding claims, **characterised in that** a release protective collar (29) which stands upright and increases in height from each of its main peaks toward the centre of the release protective collar (29) is formed on at least approximately half of the edge of the recess (22) of the locking member (21), which edge is diametrically opposite the adjacent edge of the slot opening lid (4).

14. Container according to any one of the preceding claims, **characterised in that** the first movable lid connection (13) is formed as a living hinge.

15. Container according to claim 9, **characterised in that** the container lid (2) and the slot opening lid (4) are interconnected by a second movable lid connection (14, 15) which is formed by two hinge tensioning webs (14, 15) with snap-hinge-like spring properties formed on either side of the living hinge (13).

16. Container according to claim 1, **characterised in that** it is elliptical in cross-section.

17. Container according to any one of the preceding claims, **characterised in that** a collar-like border which is elevated vertically is provided on the edge or edge collar (5) of the container lid (2) and covers the edge of the slot opening lid (4) and optionally the container lid (2), as far as the respective edge portions comprising the two movable lid connections (13; 14, 15) and the edge portion comprising the gripping projection (20), at least as far as the level of the upper face of the slot opening lid (4) when said lid is resting on the container lid (2).

## Revendications

1. Récipient de collecte et d'élimination, en particulier pour des canules, avec un couvercle de récipient (2) présentant une ouverture d'introduction (3) et un couvercle d'ouverture d'introduction (4) relié à celui-ci de façon à pouvoir être ouvert et fermé le long du bord au moyen d'au moins une première liaison de couvercle (13) mobile et pouvant être verrouillé sur celui-ci en position fermée au moyen d'un dispositif de verrouillage,
**caractérisé en ce que** le dispositif de verrouillage comprend les éléments suivants:
- un creux de verrouillage (30) en forme d'auge avec une butée de verrouillage (31) réalisée saillante dans celui-ci sur une partie de son bord, réalisé dans le couvercle de récipient (2) dans la région située entre sa partie de bord située à l'opposé de la première liaison de couvercle mobile (13) et la partie de bord voisine de l'ouverture d'introduction (3) disposée de manière correspondante à distance de celle-ci, et
- une sorte de corps de verrouillage (21) dans un évidement (22) formé dans le couvercle d'ouverture d'introduction (4) à un endroit correspondant au creux de verrouillage (30), tenu du côté du bord par une liaison de corps de verrouillage mobile (23, 24) et déplaçable manuellement vers le bas dans une position de verrouillage prédéterminée dans le creux de verrouillage (30), position dans laquelle non seulement il entoure partiellement la butée de verrouillage (31) en coopérant avec un élément de verrouillage (27) saillant sur lui en forme de crochet mais aussi il se verrouille de façon inséparable avec le côté inférieur du couvercle d'ouverture d'introduction (4) au moyen d'un élément de sécurité de verrouillage (28) réalisé sur lui en forme de languette.

2. Récipient selon la revendication 1, **caractérisé en ce que** le couvercle d'ouverture d'introduction (4) présente essentiellement la même grandeur et le même contour que le couvercle de récipient (2).

3. Récipient selon la revendication 1 ou 2, **caractérisé en ce qu'**il est prévu une configuration plane pour la face supérieure du couvercle de récipient (2) et la face inférieure du couvercle d'ouverture d'introduction (4) adaptée de façon correspondante à celle-ci.

4. Récipient selon l'une quelconque des revendications 1 à 3, **caractérisé en ce que** l'ouverture d'introduction (3) est réalisée dans le couvercle de récipient (2) en position décentrée, c'est-à-dire en position rapprochée de la première liaison de couvercle (13).

5. Récipient selon l'une quelconque des revendications précédentes, **caractérisé en ce que** sur le côté inférieur du couvercle de récipient (2) se dresse un col d'ouverture d'introduction (11) entourant l'ouverture d'introduction (3), sur le côté intérieur duquel au moins un dispositif d'extraction de canule (12) est réalisé en saillie dans l'ouverture d'introduction (3) à une distance du côté inférieur.

6. Récipient selon l'une quelconque des revendications précédentes, **caractérisé en ce que** sur le côté inférieur du couvercle d'ouverture d'introduction (4) se dresse un col en ajustement serré (16) dans une position correspondant à l'ouverture d'introduction (3), qui se trouve en ajustement collant manuellement séparable dans le col d'ouverture d'introduction (11) lorsque le couvercle d'ouverture d'introduction (4) est appliqué sur le couvercle de récipient (2).

7. Récipient selon l'une quelconque des revendications précédentes, **caractérisé en ce que** le corps de verrouillage (21) et l'évidement (22), dans lequel il est tenu, présentent des contours elliptiques adaptés l'un à l'autre et l'axe principal du corps de verrouillage (21) s'étend perpendiculairement à l'axe principal du couvercle elliptique (4) de l'ouverture d'introduction.

8. Récipient selon l'une quelconque des revendications précédentes, **caractérisé en ce que** le corps de verrouillage (21) présente approximativement la forme d'un coin analogue à un onglet sphérique avec, en position déverrouillée, une aire de section transversale croissante en hauteur sous forme triangulaire en direction verticale à partir de ses deux sommets principaux en direction du milieu.

9. Récipient selon la revendication 8, **caractérisé en ce que**, dans le corps de verrouillage (21), à l'état déverrouillé, la face inférieure, mais au contraire la face supérieure à l'état verrouillé, est plane par rapport à la face supérieure du couvercle de récipient (2).

10. Récipient selon l'une quelconque des revendications précédentes, **caractérisé en ce que** l'élément de verrouillage (27) se présente sous la forme d'une saillie de matière dirigée en forme de crochet en oblique vers le bas vers la butée de verrouillage (31) et s'étendant, à l'état déverrouillé, dans la direction de l'axe principal du corps de verrouillage (21) à partir de la face inférieure de celui-ci.

11. Récipient selon l'une quelconque des revendications précédentes, **caractérisé en ce que** l'élément de sécurité de verrouillage (28) est réalisé en forme de languette dressée verticalement au milieu sur le bord du corps de verrouillage (21) opposé à la liaison de corps de verrouillage mobile (23, 24).

12. Récipient selon l'une quelconque des revendications précédentes, **caractérisé en ce que** le corps de verrouillage (21) est assemblé au bord de l'évidement (22) sur son côté situé à l'opposé de la liaison de corps de verrouillage mobile (23, 24) par deux ponts de matière (25, 26) réalisés de manière frangible de part et d'autre de l'élément de sécurité de verrouillage (28) pendant la durée de l'utilisation du récipient.

13. Récipient selon l'une quelconque des revendications précédentes, **caractérisé en ce que**, à au moins approximativement la moitié du bord de l'évidement (22) du corps de verrouillage (21) diamétralement opposé au bord voisin du couvercle d'ouverture d'introduction (4) est formé un col dressé (29) de protection contre la séparation avec une hauteur croissante à partir de ses sommets principaux en direction du milieu du col (29) de protection contre la séparation.

14. Récipient selon l'une quelconque des revendications précédentes, **caractérisé en ce que** la première liaison de couvercle mobile (13) se présente sous la forme d'une charnière pelliculaire.

15. Récipient selon la revendication 9, **caractérisé en ce que** le couvercle de récipient (2) et le couvercle d'ouverture d'introduction (4) sont reliés l'un à l'autre par une deuxième liaison de couvercle mobile (14, 15), qui est formée par deux barrettes de serrage articulées (14, 15) avec des propriétés de ressort du type charnières à déclic réalisées de part et d'autre de la charnière pelliculaire (13).

16. Récipient selon la revendication 1, **caractérisé en ce qu'**il présente une forme de section transversale elliptique.

17. Récipient selon l'une quelconque des revendications précédentes, **caractérisé en ce qu'**il est prévu, sur le bord ou sur le col de bord (5) du couvercle de récipient (2), un rebord réalisé sous forme dressée verticalement à la manière d'un col et masquant le bord du couvercle d'ouverture d'introduction (4) et éventuellement celui du couvercle de récipient (2), jusqu'aux parties de bord respectives avec les deux liaisons de couvercle mobiles (13; 14, 15) ou jusqu'à celle avec l'onglet d'accrochage (20), au moins jusqu'au niveau de la face supérieure du couvercle d'ouverture d'introduction (4) dans la position de celui-ci appliqué sur le couvercle de récipient (2).
